# EUROPÄISCHE PATENTSCHRIFT

(11) **EP 1 380 566 B1**
(45) Veröffentlichungstag und Bekanntmachung des Hinweises auf die Patenterteilung: **11.04.2007**
(21) Anmeldenummer: 03012956.3
(22) Anmeldetag: 06.06.2003
(51) Int. Cl.: C07C 69/82

(54) **Verfahren zur Herstellung von Dimethylterephthalat**
Process of preparing dimethylterephthalat
Procédé de fabrication du dimethylterephthalat

(30) Priorität: 12.07.2002 DE 10231872
(43) Veröffentlichungstag der Anmeldung: 14.01.2004
(73) Patentinhaber: H&G Hegmanns Ingenieurgesellschaft mbH, 45881 Gelsenkirchen (DE); Sulzer Chemtech AG, 8404 Winterthur (CH)
(72) Erfinder:
(74) Vertreter: Albrecht, Rainer Harald

(56) Entgegenhaltungen:
- US-A- 3 962 315
- US-A- 5 338 882
- US-A- 5 542 372
- WYNN N P: "SEPARATE ORGANICS BY MELT CRYSTALLIZATION" CHEMICAL ENGINEERING PROGRESS, AMERICAN INSTITUTE OF CHEMICAL ENGINEERS. NEW YORK, US, Bd. 88, Nr. 3, 1. März 1992 (1992-03-01), Seiten 52-60, XP000246045 ISSN: 0360-7275

## Beschreibung

Die Erfindung betrifft ein Verfahren zur Herstellung von Dimethylterephthalat (DMT), bei dem ein p-Xylol und p-Toluylsäuremethylester (PTE) enthaltendes Gemisch in flüssiger Phase in Gegenwart eines Schwermetallkatalysators mit sauerstoffhaltigem Gas oxidiert wird, das Oxidationsprodukt nach einer Strippung von p-Xylol und leicht siedenden Bestandteilen mit Methanol zu einem Rohester verestert wird und aus dem Rohester das DMT isoliert wird.

Dimethylterephthalat (DMT) kann vollständig oder teilweise zur Terephtalsäure (TPA) hydrolisiert werden. DMT und TPA sind wichtige Rohstoffe für die Herstellung von Polyestern, Polyamiden, verschiedenen anderen Estern und chemischen Produkten. Für die Herstellung von Polyesterfasern werden DMT und TPA in hoher Reinheit benötigt. Das eingangs beschriebene Verfahren zur Herstellung von DMT wird in der Literatur als Katzschmann-Verfahren oder Witten-Verfahren bezeichnet (Ullmanns Enzyklopädie der technischen Chemie, 4. Auflage, Band 22, Verlag Chemie 1982, Seiten 530 und 531). Bei diesem Verfahren werden zunächst p-Xylol und p-Toluylsäuremethylester (PTE) mit Sauerstoff enthaltenden Gasen in Gegenwart von halogenfreien Schwermetallkatalysatoren, in der Regel Co/Mn-Mischungen, oxidiert. PTE wird dem Reaktionsgemisch im Überschuss zugesetzt, da es als Transport- und/oder Lösemittel für feste hochschmelzende Oxidationsprodukte dient, und aus nachfolgenden Verfahrensstufen bzw. Reaktoren in den Oxidationsreaktor zurückgeführt. In einer der Oxidationsstufe nachfolgenden Veresterungskolonne wird das Oxidationsprodukt mit praktisch allen Zwischen- und Nebenprodukten der Oxidation verestert. Der aus der Veresterungskolonne abgezogene Rohester enthält im Wesentlichen PTE, DMT und Isomere von DMT sowie eine Mehrzahl von veresterten Zwischen- und Nebenprodukten. Diese Zwischen- und Nebenprodukte enthalten zunächst Wertstoffe, die wieder zu DMT umgesetzt werden können und hierzu zur Ausbeuteerhöhung in den Prozess zurückgeführt werden. Hierbei handelt es sich insbesondere um aromatische Alkohole, wie Hydroxymethylbenzoesäuremethylester (HMBME), um aromatische Aldehyde, wie Terephtalaldehydsäuremethylester (TAE) oder um in der Veresterung nicht umgesetzte Säuren wie p-Toluylsäure (PTS) und Monomethylterephthalat (MMT). HMBME und TAE sind veresterte Oxidationsprodukte, die aus der Oxidation von p-Xylol bzw. PTE resultieren. Die Zwischen- und Nebenprodukte enthalten aber auch unerwünschte Stoffe, die insbesondere bei Anreicherung die Verfahrensstufen nachteilig beeinflussen und die Ausbeute beeinträchtigen können. Darunter fallen beispielsweise Benzoesäuremethylester (BME) sowie mehrkernige aromatische Substanzen wie Diphenyle und Triphenyle. Besondere nachteilige Substanzen sind Trimellitsäure (TMS), Trimellitsäuremonomethylester (TMME) und der daraus durch Veresterung entstehende hochsiedende Trimellitsäuretrimethylester (TMT). Die Trimellitsubstanzen wirken als Katalysatorinhibitoren und beeinträchtigen insoweit die Oxidation, als schlechte Ausbeuten resultieren oder aber die Oxidationsreaktion nicht gestartet wird, gehemmt wird oder abbricht. Weitere unerwünschte Stoffe im Rohester sind stark gelbfärbende Substanzen, die in der Literatur auch als Gelböle bezeichnet werden und die Qualität des Zielproduktes bereits in extrem geringen Konzentrationen stark beeinträchtigen.

Bei einem aus DE-A 39 04 586 bekannten Verfahren zur Herstellung von DMT wird der Rohester destillativ in eine PTE-reiche Fraktion, eine aus Dimethylphthalaten (DMP) bestehende DMP-Fraktion sowie eine schwer siedende Rückstandsfraktion aufgetrennt. Die PTE-reiche Fraktion wird in die Oxidationsstufe zurückgeführt. Die DMP-Fraktion, die das DMT, Isomere des DMT und weniger als 1 Gew.% sonstige Begleitstoffe enthält, wird durch Lösungsmittel-Umkristallisation in Methanol soweit gereinigt, dass die Anteile an HBME und TAE zusammen weniger als 200 ppm im Endprodukt betragen. Die zweistufig ausgeführte Lösungsmittelkristallisation erfordert einen erheblichen anlagentechnischen Aufwand, da große Stoffströme filtriert werden müssen. Wenn man bedenkt, das zwei Teile Methanol für ein Teil DMP gebraucht werden, ist einsichtig, dass auch große Energiemengen zur Destillation aufgewendet werden müssen, damit das Lösungsmittel Methanol eine für den erneuten Einsatz in der Kristallisation genügende Qualität erreicht.

Aus der Druckschrift US 5 542 372 ist ein Verfahren zur Herstellung von DMT bekannt, bei dem p-Xylol und p-Toluylsäuremethylester (PTE) mit Luft unter der Anwesenheit eines Kobalt/Magnesium-Acetatkatalysators oxidiert wird. Das hierbei erhaltene Gemisch wird anschließend mit Methanol zu DMT, PTE und weiteren Komponenten verestert. Hieraus wird destillativ ein Roh-DMT gewonnen, welches nachfolgend mit Hilfe einer Fallfilmkristallisation aufgearbeitet wird. Eine weitere Aufbereitung oder Reinigung von DMT wird nicht beschrieben.

Der Erfindung liegt die Aufgabe zugrunde, ein Verfahren zur Herstellung von DMT anzugeben, mit dem ein Produkt hoher Reinheit energetisch günstig und mit geringem anlagentechnischen Aufwand hergestellt werden kann.

Gegenstand der Erfindung und Lösung dieser Aufgabe ist ein Verfahren zur Herstellung von Dimethylterephthalat (DMT) mit folgenden Verfahrensschritten:
1.1) ein p-Xylol und p-Toluylsäuremethylester (PTE) enthaltendes Gemisch wird in flüssiger Phase in Gegenwart eines Schwermetallkatalysators mit sauerstoffhaltigem Gas oxidert;
1.2) das Oxidationsprodukt wird nach einer Strippung von p-Xylol und leicht siedenden Bestandteilen mit Methanol zu einem Rohester verestert;
1.3) aus dem Rohester wird eine im Wesentliche aus Benzolsäuremethylester (BME) und p-Toluylsäuremethylester (PTE) bestehende BME/PTE-Fraktion destillativ abgetrennt;
1.4) aus der verbleibenden Rohesterrückstandsfraktion werden Hochsieder, die den Schwermetallkatalysator und gelbfärbende Substanzen umfassen, in einer weiteren Trennstufe als Sumpfprodukt abgetrennt;
1.5) das Destillat aus dieser Trennstufe wird einer Schmelzkristallisation unterworfen, in der Terephtalaldehyd (TAE) und Hydroxymethylbenzosäuremethylester (HMBME) als flüssige Phase abgetrennt werden und dadurch eine aus Dimethylphthalaten (DMP) bestehende Fraktion isoliert wird, die das DMT, Isomere des DMT und weniger als 1 Gew.-% sonstige Begleitstoffe enthält und als Kristallisat anfällt;
1.6) die DMP-Fraktion wird durch eine weitere Schmelzkristallisation in DMT und mindestens eine die Isomere des DMT sowie die Begleitstoffe enthaltende Fraktion aufgetrennt.

Die Schmelzkristallisation wird vorzugsweise als Fallfilmkristallisation betrieben, bei der die DMP-Fraktion flüssig auf den Kopf eines stehenden Rohrbündels aus gekühlten Rohren aufgegeben wird, wobei an den gekühlten Rohrflächen DMT auskristallisiert und die Isomere des DMT als niedrig schmelzende Fraktion in einer Vorlage aufgefangen werden. Das Rohrbündel weist zweckmäßig eine Länge zwischen 10 m und 40 m auf, wobei Längen von etwa 20 m bevorzugt sind. Die Rohre besitzen einen Durchmesser von weniger 100 mm und sind in einem Mantel zwischen Rohrböden angeordnet. Oberseitig ist eine Aufgabevorrichtung für eine gleichmäßige Flüssigkeitsverteilung vorgesehen. Es ergeben sich günstige Verteilungen, wenn die pro Bündel aufgegebene Flüssigkeit 15 m3 pro Stunde nicht übersteigt. Selbst bei sehr großen Leistungen ergeben sich überschaubare und einfach zu betreibende Anlagen. Das Rohrbündel wird von außen gekühlt, wobei die Kühlmitteltemperatur auf einen Gemisch spezifisch festgelegten Wert unterhalb der Schmelztemperatur der abzuscheidenden höher schmelzenden Substanz, in diesem Fall DMT, eingestellt wird. Das DMT kristallisiert auf den gekühlten Rohroberflächen aus, während eine kleine Menge als Rest der aufgegebenen Flüssigkeit die ablaufende niedrig schmelzende Fraktion bildet, die neben den Isomeren und den Begleitstoffen auch noch eine gewisse Menge DMT enthält. Die niedrig schmelzende, ablaufende Fraktion wird in einer Vorlage aufgefangen und später separat aufgearbeitet, wobei das dabei zugrückgewonnene DMT in den Prozess zurückgeführt und die Isomere und Begleitstoffe ausgeschleust werden. Für die Aufarbeitung der niedrig schmelzenden Fraktion eignet sich insbesondere eine statische Schmelzkristallisation.

Das bei der Fallfilmkristallisation auf den gekühlten Rohrflächen haftende Kristallisat kann zur Verbesserung der Produktreinheit vor dem Abschmelzen leicht erwärmt werden. Dabei treten niedrig schmelzende Bestandteile aus und tropfen in eine Vorlage ab. Die abtropfende Flüssigkeit wird vorzugsweise separat aufgefangen und wieder in den Prozess zurückgegeben. Sie kann aber auch z. B. mit der niedrig schmelzenden Fraktion aufgearbeitet werden. Die beschriebene Behandlung und die dabei auftretende Flüssigkeitsabsonderung wird als "Schwitzen" bezeichnet.

Das nach dem Schwitzen erhaltene Kristallisat wird abgeschmolzen und in einer Vorlage gesammelt. Sofern das Produkt die geforderte Reinheit noch nicht besitzt, wird die Schmelzkristallisation wiederholt. Die in mehreren Zyklen hergestellten verschieden reinen Produktfraktionen werden zweckmäßig in separaten Vorlagen gelagert, um Rückvermischungen zu vermeiden. Die Anzahl der notwendigen Zyklen, auch Stufen genannt, erfordert eine entsprechende Anzahl von Vorlagen.

Da TAE und HMBME in hoch reinem DMT unerwünscht sind und Konzentrationen von maximal 10 ppm toleriert werden, wird die Konzentration dieser Stoffe in der DMP-Ausgangsfraktion abgesenkt, um die Anzahl der Reinigungszyklen in der Schmelzkristallisation zu reduzieren. Vorzugsweise sollen die Begleitstoffe in der DMP-Ausgangsfraktion nicht mehr als 500 ppm betragen. Die Absenkung des Isomerengehaltes auf die maximal zulässige Konzentration in DMT hat sich als wenig problematisch erwiesen. Wenn die für die Absenkung der übrigen Begleitstoffe notwenigen Zyklen durchgeführt sind, ist auch die zulässige Konzentration der Isomere erreicht, wenn das in der Oxidationsstufe eingesetzte Xylol nicht mehr als 2 % an Isomeren enthält. Als weitaus kritischste Stoffgruppe erweisen sich die gelbfärbenden Substanzen, deren Abreicherung durch Schmelzkristallisation wesentlich problematischer ist als durch Lösungskristallisation. Die maximal zulässige Konzentration im hochreinen DMT ist allerdings auch so gering, dass sie nur durch Messen einer Extinktion von kurzwelligem Licht im DMT-Endprodukt bestimmt werden kann. Die Einhaltung der maximal zulässigen Konzentration ist mit einer wirtschaftlichen Anzahl von Reinigungszyklen der Schmelzkristallisation nur möglich, wenn die gelbfärbenden Substanzen vor der Schmelzkristallisation quantitativ, also vollständig, aus dem Rohester entfernt werden.

Die leichter siedenden Fraktionen des Rohesters, die PTE sowie Wertstoffe darstellende Zwischen- und Nebenprodukte enthalten, werden in an sich bekannter Weise in die Oxidationsstufe des Prozesses zurückgeführt. Aus schwerer siedenden Fraktionen des Rohesters, welche die gelbfärbenden Substanzen enthalten, werden Wertstoffe zurückgewonnen sowie ein die gelbfärbenden Substanzen sowie Katalysatorgifte enthaltender Stoffstrom isoliert und ausgeschleust.

Bei der Aufarbeitung des Rohesters kann eine Schmelzkristallisation ebenfalls vorteilhaft eingesetzt werden, denn im Gegensatz zum Siedeverhalten von Terephtalaldehydsäuremethylester (TAE) und Hydroxymethylbenzoesäuremethylester (HMBME), die eine nur wenig niedrigere bzw. wenig höhere Siedetemperatur als DMT haben, sind deren Schmelzpunkte untereinander ähnlich aber wesentlich niedriger als der von DMT. Dadurch lassen sich TAE und HMBME mit geringem Energieaufwand durch Schmelzkristallisation von DMT trennen, während bei einer destillativen Trennung extreme Rückläufe und hohe DMT-Beimengungen mit dem damit verbundenen Aufwand und Nachteilen erforderlich sind, um eine einigermaßen zufrieden stellende Trennschärfe zu erreichen. Gemäss einer bevorzugten Ausführung der Erfindung wird aus dem Rohester zunächst eine im Wesentlichen aus Benzoesäuremethylester (BME) und p-Toluylsäuremethylester (PTE) bestehende BME/PTE-Fraktion destillativ abgetrennt. Aus der verbleibenden Rohesterrückstandsfraktion werden in einem zweiten Trennschnitt Hochsieder, die den Schwermetallkatalysator und die gelbfärbenden Substanzen umfassen, als Sumpfprodukt abgetrennt. Schließlich wird das Destillat aus dieser Trennstufe einer Schmelzkristallisation unterworfen, in der Terephtalaldehydsäuremethylester (TAE) und Hydroxymethylbenzoesäuremethylester (HMBME) als flüssige Phase abgetrennt werden. Das abgeschmolzene DMT-Kristallisat (DMP-Fraktion) besitzt nur noch eine schwache gelbliche Färbung und eignet sich als Vorprodukt für die nachfolgende Endreinigung durch Schmelzkristallisation. Bei sehr hohen Qualitätsansprüchen kann die DMP-Fraktion noch einmal aufdestilliert werden, wobei als Sumpfprodukt eine sehr geringe Menge DMP anfällt, das mit gelbfärbenden Substanzen angereichert ist und sich beispielsweise für eine Aufarbeitung in der Methanolyse eignet. Das als Destillat anfallende DMP ergibt bereits nach wenigen Reinigungszyklen in der abschließenden Schmelzkristallisation ein DMT überlegener Qualität. Besonders günstig wirkt sich dabei aus, dass die Schmelzkristallisation zur Abtrennung von TAE bzw. HMBME einerseits und zur Reinigung der DMP-Fraktion andererseits chargenweise in derselben Fallfilmkristallisationsanlage durchgeführt werden können.

Eine weitere, unter die erfindungsgemäße Lehre fallende Ausgestaltung ist in dem Unteranspruch 4 beschrieben.

Im Folgenden wird die Erfindung anhand einer lediglich ein Ausführungsbeispiel darstellenden Zeichnung erläutert.

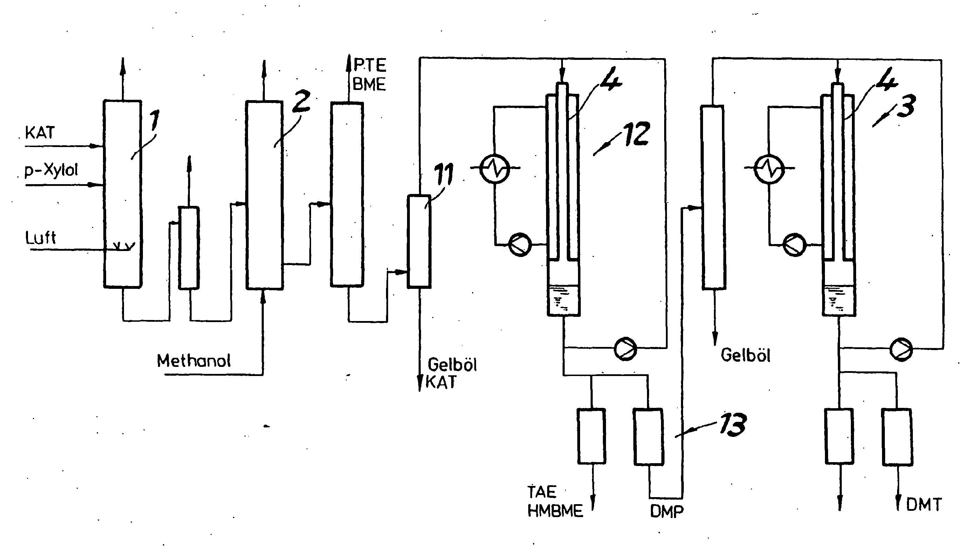

Die einzige Figur zeigt ein Verfahren zur Herstellung von Dimethylterephthalaten (DMT).

Bei dem in der einzigen Figur dargestellten Verfahren wird ein p-Xylol und PTE enthaltendes Gemisch in flüssiger Phase in Gegenwart eines Schwermetallkatalysators in mindestens einem Oxidationsreaktor 1 mit sauerstoffhaltigem Gas oxidiert. Das Oxidationsprodukt wird nach einer Strippung von p-Xylol und leicht siedenden Bestandteilen mit Methanol in einer Veresterungskolonne 2 zu einem Rohester RE verestert. Aus dem Rohester RE werden gelbfärbende Substanzen quantitativ abgetrennt und es wird eine aus Dimethylphthalaten bestehende DMP-Fraktion isoliert, die das DMT, Isomere des DMT und weniger als 1 Gew.% sonstige Begleitstoffe enthält. Die DMP-Fraktion wird anschließend durch Schmelzkristallisation 3 in das DMT und mindestens eine die Isomere des DMT sowie die Begleitstoffe enthaltende Fraktion aufgetrennt. Die Schmelzkristallisation wird als Fallfilmkristallisation betrieben, bei der die DMP-Fraktion flüssig auf den Kopf eines stehenden Rohrbündels 4 aus gekühlten Rohren aufgegeben wird. An den gekühlten Rohrflächen kristallisiert DMT aus, während eine niedrig schmelzende Fraktion, welche die Isomere des DMT und die Begleitstoffe enthält, in einer Vorlage aufgefangen werden. Um die Produktreinheit des Kristallisates zu verbessern, wird das auf den gekühlten Flächen haftende Kristallisat vor dem Abschmelzen um 0,5 bis 10°C leicht erwärmt. Dabei treten niedrig schmelzende Bestandteile aus dem Kristallisat aus und tropfen in eine separate Vorlage ab. Wenn die gewünschte Reinheit des Kristallisates noch nicht erreicht ist, wird der Vorgang wiederholt. Bei der Aufarbeitung des Rohesters werden leichter siedende Fraktionen des Rohesters, die PTE sowie Wertstoffe darstellende Zwischen- und Nebenprodukte enthalten, in die Oxidationsstufe 1 zurückgeführt. Aus schwerer siedenden Fraktionen des Rohesters, welche die gelbfärbenden Substanzen enthalten, werden Wertstoffe zurückgewonnen sowie ein die gelbfärbenden Substanzen sowie Katalysatorgifte enthaltener Stoffstrom isoliert und ausgeschleust.

Bei dem in der einzigen Figur dargestellten erfindungsgemäßen Verfahren werden aus dem Rohester eine Benzoesäuremethylester und p-Toluylsäuremethylester enthaltende leicht siedende BME/PTE-Fraktion destillativ abgetrennt. Aus der verbleibenden Rohesterrückstandsfraktion werden durch eine Flashdestillation 11 Hochsieder, die den Schwermetallkatalysator KAT und gelbfärbende Substanzen (Gelböl) umfassen, als Sumpfprodukt abgetrennt. Das Destillat aus dieser Trennstufe wird einer Schmelzkristallisation 12 unterworfen, in der TAE und HMBME als niedrig schmelzende Phase abgetrennt werden. Das Kristallisat besitzt eine noch leichte gelbliche Färbung und eignet sich als Vorprodukt für eine nachfolgende Endreinigung durch Schmelzkristallisation. Bei hohen Qualitätsanforderungen wird es in einer Kolonne 13 noch einmal aufdestilliert, wobei als Sumpfprodukt eine sehr kleine Menge DMP anfällt, das mit Gelböl angereichert ist und sich gut für eine Aufarbeitung durch Methanolyse eignet. Das als Destillat anfallende DMP wird der DMP-Fraktion einer weiteren Schmelzkristallisation zugeführt und in das DMT sowie mindestens eine die Isomere und die Begleitstoffe enthaltende Fraktion aufgetrennt. Dabei fällt ein hochreines DMT in der Qualität DMT-p an.

## Patentansprüche

1. Verfahren zur Herstellung von Dimethylterephthalat (DMT) mit folgenden Verfahrensschritten
1.1) ein p-Xylol und p-Toluylsäuremethylester (PTE) enthaltendes Gemisch wird in flüssiger Phase in Gegenwart eines Schwermetallkatalysators mit sauerstoffhaltigem Gas oxidiert;
1.2) das Oxidationsprodukt wird nach einer Strippung von p-Xylol und leicht siedenden Bestandteilen mit Methanol zu einem Rohester verestert;
1.3) aus dem Rohester wird eine im Wesentlichen aus Benzolsäuremethylester (BME) und p-Toluylsäuremethylester (PTE) bestehende BME/PTE-Fraktion destillativ abgetrennt;
1.4) aus der verbleibenden Rohesterrückstandsfraktion werden Hochsieder, die den Schwermetallkatalysator und gelbfärbende Substanzen umfassen, in einer weiteren Trennstufe als Sumpfprodukt abgetrennt;
1.5) das Destillat aus dieser Trennstufe wird einer Schmelzkristallisation unterworfen, in der Terephtalaldehyd (TAE) und Hydroxymethylbenzosäuremethylester (HMBME) als flüssige Phase abgetrennt werden und **dadurch** eine aus Dimethylphthalaten (DMP) bestehende Fraktion isoliert wird, die das DMT, Isomere des DMT und weniger als 1 Gew.-% sonstige Begleitstoffe enthält und als Kristallisat anfällt;
1.6) die DMP-Fraktion wird durch eine weitere Schmelzkristallisation in DMT und mindestens eine die Isomere des DMT sowie die Begleitstoffe enthaltende Fraktion aufgetrennt.

2. Verfahren nach Anspruch 1, **dadurch gekennzeichnet, dass** die Schmelzkristallisation als Fallfilmkristallisation betrieben wird, bei der die DMP-Fraktion flüssig auf den Kopf eines stehenden Rohrbündels aus gekühlten Rohren aufgegeben wird, wobei an den gekühlten Rohrflächen DMT auskristallisiert und die Isomere des DMT in einer Vorlage aufgefangen werden.

3. Verfahren nach Anspruch 2, **dadurch gekennzeichnet, dass** das auf den gekühlten Flächen haftende Kristallisat zur Verbesserung der Produktreinheit vor dem Abschmelzen leicht erwärmt wird, wobei niedrig schmelzende Bestandteile aus dem Kristallisat austreten und abtropfen.

4. Verfahren nach Anspruch 1 bis 3, **dadurch gekennzeichnet, dass** die bei der Schmelzkristallisation zur Isolierung der DMP-Fraktion anfallende niedrig schmelzende Phase durch Rektifikation und/oder eine weitere Schmelzkristallisation aufgetrennt wird in Wertstoffe enthaltende Fraktionen und Fraktionen, welche die gelbfärbenden Substanzen enthalten und ausgeschleust werden.

## Claims

1. A process for producing dimethyl terephthalate (DMT) comprising the following process steps:
1.1) a mixture containing p-xylene and p-toluic acid methyl ester (PTE) is oxidised in the liquid phase with oxygen-containing gas in the presence of a heavy metal catalyst;
1.2) the oxidation product is esterified with methanol to form a raw ester after stripping p-xylene and light-boiling components;
1.3) a BME/PTE fraction substantially containing benzoic acid methyl ester (BME) and p-toluic acid methyl ester (PTE) is distillatively separated from the raw ester;
1.4) high-boiling components comprising the heavy metal catalyst and yellow-colouring substances are separated from the remaining raw ester residue fraction in a further separating stage as sump product;
1.5) the distillate from this separating stage is subjected to melt crystallisation in which terephthalaldehyde (TAE) and hydroxymethyl benzoic acid methyl ester (HMBME) are separated as liquid phase and a fraction consisting of dimethyl phthalates (DMP) is thereby isolated, said fraction containing the DMT, isomers of DMT and less than 1 wt.% of other accompanying substances and is present as crystallisate;
1.6) the DMP fraction is separated by a further melt crystallisation into at least one fraction containing the isomers of DMP and the accompanying substances.

2. The process according to claim 1, **characterised in that** the melt crystallisation is operated as falling-film crystallisation wherein the DMP fraction is fed in liquid form to the head of a vertical tube bundle of cooled tubes, wherein DMT crystallises out on the cooled tube surfaces and the isomers of DMT are collected in a receiving flask.

3. The process according to claim 2, **characterised in that** the crystallisate adhering to the cooled surfaces is heated slightly before the melting to improve the product purity, wherein low-melting components leave the crystallisate and drip off.

4. The process according to claim 1 to 3, **characterised in that** the low-melting phase which accumulates during the melt crystallisation to isolate the DMP fraction is separated by rectification and/or a further melt crystallisation to give fractions containing valuable substances and fractions which contain yellow-colouring substances and are discharged.

## Revendications

1. Procédé de préparation de diméthyltéréphtalate (DMT) par les étapes opératoires suivantes
1.1) un mélange contenant du p-xylol et p-du méthylester d'acide toluylique (PTE) est oxydé en phase liquide en présence d'un catalyseur de métaux lourds avec du gaz contenant de l'oxygène ;
1.2) le produit d'oxydation, après dépouillage du p-xylol et de composantes bouillant facilement, est estérifié en ester brut ;
1.3) à partir de l'ester brut, une fraction de BME/PTE composée substantiellement de méthylester d'acide benzoïque (BME) et de p-méthylester d'acide toluylique (PTE) est isolée par distillation ;
1.4) à partir de la fraction de résidu d'ester brut restante, des produits à haute ébullition englobant le catalyseur de métal lourd et des substances de coloration en jaune sont isolés dans une autre étape d'isolation sous forme de produit liquide ;
1.5) le distillat issu de cette étape d'isolation est soumis à une cristallisation de coulée, pendant laquelle sont isolés de l'aldéhyde de téréphtalate (TAE) et de l'hydroxyméthyl-méthylester d'acide benzoïque (HMBME) en phase liquide et est ainsi isolée une fraction composée de diméthylphtalate (DMP) qui contient le DMT, des isomères du DMT et moins de 1 % en poids d'autres substances d'accompagnement et se présente sous forme d'un cristallisat ;
1.6) la fraction de DMP, par une autre cristallisation de coulée, est divisée en DMT et en au moins une fraction contenant les isomères du DMT ainsi que les substances d'accompagnement.

2. Procédé selon la revendication 1, **caractérisé en ce que** la cristallisation de coulée est effectuée sous forme d'une cristallisation de film précipité, lors de laquelle la fraction de DMP est versée sous forme liquide sur le sommet d'un faisceau de tubes dressé fait de tubes refroidis, le DMT se cristallisant sur les surfaces de tuyaux refroidies et les isomères du DMT étant récupérés dans un récipient.

3. Procédé selon la revendication 2, **caractérisé en ce que** le cristallisat adhérant sur les surfaces refroidies est légèrement chauffé pour améliorer la pureté du produit avant la fusion, tandis que des composantes à basse fusion sortent et gouttent du cristallisat.

4. Procédé selon les revendications 1 à 3, **caractérisé en ce que** la phase à basse fusion apparaissant lors de la cristallisation de coulée pour isoler la fraction de DMP est isolée par rectification et/ou une autre cristallisation de coulée en substances valorisables et en fractions qui contiennent les substances de coloration en jaune et qui sont rejetées.
